Europäisches Patentamt

European Patent Office

Office européen des brevets

(19)

(11) Numéro de publication : **0 675 197 A1**

# (12) DEMANDE DE BREVET EUROPEEN

(21) Numéro de dépôt : 95870021.3

(22) Date de dépôt : 14.03.95

(51) Int. Cl.⁶ : **C12N 9/96,** C12N 11/00, G01N 33/543

(30) Priorité : 28.03.94 BE 9400331

(43) Date de publication de la demande :
04.10.95 Bulletin 95/40

(84) Etats contractants désignés :
DE FR GB IT

(71) Demandeur : **ANDA BIOLOGICALS S.A.**
37, rue de la Course
F-67067 Strasbourg Cédex (FR)

(72) Inventeur : **Vu Khue, Nguyen**
21, rue du Paridis,
**Wittersheim**
**F-67670 Mommenheim (FR)**
Inventeur : **Poindron, Philippe**
rue André Malraux 14,
**Plobsheim**
**F-67400 Illkirch (FR)**
Inventeur : **Maes, Roland**
**Bildhauerhof, 34,**
**Rosheim**
**F-67190 Mutzig (FR)**

(74) Mandataire : **Van Malderen, Michel et al**
**Office van Malderen**
**Place Reine Fabiola 6/1**
**B-1080 Bruxelles (BE)**

(54) **Support solide fixant l'acétylcholinestérase et/ou un récepteur à acétylcholinestérase stabilisé et procédé pour son obtention.**

(57) La présente invention concerne un support solide fixant l'acétylcholinestérase et/ou un récepteur à acétylcholinestérase, recouvert d'un film protecteur à base de gélatine et/ou d'albumine et à base de tréhalose.
La présente invention concerne également le procédé d'obtention dudit support solide.

FIG. 1

## Objet de l'invention.

La présente invention concerne un support solide fixant l'acétylcholinestérase et/ou un récepteur à acétylcholinestérase stabilisé, ainsi que son procédé d'obtention.

## Arrière-plan technologique à la base de l'invention.

Les récents développements en biologie moléculaire et en biochimie ont permis l'utilisation de nombreux composants à activité biologique dans des procédés de synthèse, de séparation et/ou de diagnostic.

Parmi ces composants à activité biologique, les enzymes se sont révélés de bons catalystes par leur forte spécificité pour leur substrat, et leur forte activité catalytique pouvant fonctionner dans des conditions très douces.

Cependant, ces procédés nécessitent souvent une modification de la température, du pH, de la concentration du milieu extérieur, une dessiccation des supports, etc., ce qui occasionne souvent la dénaturation ou la destruction de ces enzymes.

Les enzymes sont caractérisées par une conformation tridimensionnelle spécifique obtenue par l'agencement d'acides aminés et les liens pouvant exister entre ces éléments.

La dénaturation est un procédé qui induit une modification mineure ou majeure de cette conformation, sans spécialement perturber la séquence des différents éléments.

Cette dénaturation peut provoquer une diminution, voire une inactivation complète, de l'activité biologique de ces composants.

Parmi les enzymes et récepteurs enzymatiques dont la dénaturation spontanée à température ambiante est très rapide se trouve l'acétylcholinestérase ainsi que le récepteur membranaire de l'acétylcholinestérase. Ces deux entités présentent un grand intérêt analytique et diagnostique pour la détection de certains types de pesticides, pour l'évaluation du taux sanguin de certains médicaments et pour le diagnostic de certaines maladies (myasthénie musculaire, maladie d'Alzheimer, ...).

Les pesticides couramment utilisés sont de divers types, ayant tous une action inhibitrice propre sur un site réactionnel bien défini. Certains d'entre eux, appartenant au groupe des organophosphores et des carbamates, inhibent la cholinestérase, ce qui entraîne des désordres au niveau de la transmission de l'influx nerveux.

La détection des traces de pesticides contaminant les aliments destinés à la consommation animale et humaine (vins, céréales, légumes, jus de fruits, fruits, aliments pour bébés, ...) est pour l'instant basée sur la technique de chromatographie en phase gazeuse. Cette technique très performante est malheureusement extrêmement difficile à mettre en oeuvre, longue et coûteuse. De plus, elle n'autorise pas une détermination globale des pesticides incriminés, mais fait de façon directe une analyse détaillée des échantillons soumis à analyse.

## Etat de la technique.

Différentes techniques ont été proposées pour stabiliser les enzymes, c'est-à-dire réduire ou empêcher leur dénaturation par dessiccation, modification du pH et/ou de la pression, action de protéases, ... .

Les procédés de stabilisation des enzymes présentes dans une phase liquide sont bien connues depuis plusieurs années (Enzyme Stabilisation, Gianfreda and Scarfi, Mol. Cell Biochemistry 1991, 110, pp 97-128).

Parmi les différentes techniques qui sont satisfaisantes, on peut citer l'immobilisation sur phase solide, l'adjonction d'additifs, le couplage à d'autres composants, la réticulation par des agents bifonctionnels.

Certaines enzymes sont stabilisées par des sucres à l'état liquide, mais en général, les sucres ne stabilisent normalement ces enzymes sous forme soluble qu'en dessous de la température de congélation de l'eau, préférablement à -20 °C ou encore moins.

La demande de brevet FR-2677373 décrit une trousse de détection de pesticides de la famille des organophosphores et/ou des carbamates en milieu aqueux par une détermination colorimétrique connue depuis longtemps (A New and Rapid Colorimetric Determination of Acetylcholinesterase Activity, Ellman et al. Biochem. Pharmacology, 1961, 7, pp 88-95). L'élément de détection des pesticides consiste en une enzyme de la famille des cholinestérases fixée selon un procédé complexe à un support solide par l'intermédiaire d'une matrice protéique. La matrice protéique est de la gélatine dans laquelle l'enzyme est noyée, puis réticulée par un agent bifonctionnel tel que la glutaraldéhyde. Le support solide consiste en un bâtonnet fixé sur un bouchon et plongeant dans un tube test contenant un milieu aqueux constitué d'eau physiologique tamponnée à neutralité par un tampon phosphate ainsi qu'un bactériostatique. Cette solution garde perpétuellement mouillée l'enzyme fixée de façon covalente au support solide par la gélatine enrobant le bâtonnet terminé par une hélice (Hélice immunostick maxisorp NUNC).

La fabrication de ces hélices enrobées de gélatine incorporant et fixant l'enzyme, nécessite que les hélices soient maintenues à 37 °C dans une solution aqueuse de gélatine (incorporant l'enzyme) à 5%, puis qu'elles soient séchées. Elles sont ensuite trempées dans un bain de glutaraldéhyde à 1%, rincées à l'eau distillée, plongées dans une solution de glycine pour inactiver la glutaraldéhyde, à nouveau rincées à l'eau distillée et enfin plongées de façon définitive dans un tube test contenant une solution aqueuse tamponnée à pH 7. La température de stockage est préférentiellement 4 °C.

Cependant, dans certaines applications, il est intéressant d'obtenir une enzyme attachée à la phase solide sous forme sèche, par exemple dans des tests de diagnostic.

Il est connu que certaines protéines immobilisées sur une phase solide acquièrent une stabilisation souvent supérieure à celle observée lorsqu'elles sont gardées sous forme solubilisée.

L'immobilisation de ces protéines peut être réalisée par fixation de la phase solide par liaisons covalentes, interactions adsorptives, capture de la protéine dans un gel, sur des fibres ou des grains, couplage par des réactifs bifonctionnels et/ou encapsulation dans des microcapsules ou des membranes.

La demande de brevet WO92/08134 décrit le formation de tels grains de gélatine pouvant enrober des particules magnétiques et qui possèdent des groupes fonctionnels libres sur lesquels il est possible de greffer des anticorps. Ces particules présentent l'avantage de pouvoir être plus facilement utilisées dans des milieux liquides pour la séparation de substances biologiques.

Le brevet FR-2677373 susmentionné fait appel à une technique d'immobilisation similaire.

La demande de brevet EP-140489 décrit des procédés de stabilisation de substances biologiques sur une phase solide. Lesdites substances biologiques sont caractérisées par une activité immunologique et sont choisies parmi le groupe constitué par les anticorps, les antigènes et les haptènes (hormones, globuline, antigènes viraux, vitamines, ...). Le procédé de stabilisation de ces substances fixées à une phase solide, comprend l'immersion de ces substances immuno-actives dans une solution comprenant des sucres ou des protéines. Les sucres décrits dans cette demande de brevet sont le ribose, le glucose, le fructose, le mannose, le galactose, le maltose, le lactose, le sucrose, les oligosaccharides ou les polysaccharides tels que le dextrane ou la dextrine. Parmi les protéines décrites, on mentionne la sérum-albumine bovine, humaine et de mouton, ainsi que la gélatine.

Cependant, ce document ne décrit ni ne suggère la stabilisation de molécules présentant une activité enzymatique.

En effet, les enzymes sont caractérisées par une extraordinaire labilité. Par exemple, l'acétylcholinestérase extraite du poisson torpille perd généralement toute activité enzymatique lorsqu'elle est conservée et séchée à 4 °C pendant plus de 10 jours.

Le brevet US-4,324,858 décrit une trousse de détection de pesticides comportant une cholinestérase stabilisée par l'adjonction d'un tampon switteronique et séchée sur un support solide de papier sous vide.

Des sucres sont également fréquemment utilisés pour stabiliser des enzymes séchées.

Il est connu que certains sucres sont également d'excellents agents de stabilisation d'enzymes lorsque celles-ci sont séchées à température ambiante. Il a été récemment montré que le tréhalose (α-D glucopyranosyl-α-D glucopyranoside) est un excellent agent stabilisant pour les enzymes séchées à température ambiante (Extraordinary Stability of Enzymes Dried in Trehalose, C.Colaço et al. Biotechnology 1992, 9, pp 1007-1013).

La demande de brevet européen EP-A-0192320 décrit que les agents protecteurs de l'activité immunologique des anticorps ne sont pas satisfaisants pour les enzymes.

Ce document décrit un procédé de stabilisation d'une enzyme conjuguée à un anticorps suspendu dans un milieu de protection composé d'un sucre et d'une protéine.

Ce document mentionne que l'enzyme est libre, c'est-à-dire qu'elle n'est pas fixe sur une phase solide, et que les résultats les plus satisfaisants sont obtenus avec le lactose et le tréhalose, et que de l'ovalbumine présente à une concentration de 1 % augmente encore la stabilité induite par le sucrose.

Cependant, les enzymes décrites dans ce document sont des enzymes utilisées dans les tests immuno-enzymatiques, c'est-à-dire la peroxydase, la phosphatase, la catalase et la glucosoxydase.

Il est important de noter que toutes les enzymes ne se ressemblent pas, et qu'une protection adéquate pour préserver l'activité enzymatique d'une phosphatase, une catalase ou une peroxydase peut ne pas être suffisante pour protéger l'activité enzymatique d'une enzyme très labile telle qu'une estérase, en particulier une acétylcholinestérase ou le récepteur d'une enzyme estérase telle que l'acétylcholinestérase.

## Buts de l'invention.

La présente invention vise à obtenir un support solide fixant l'acétylcholinestérase et/ou un récepteur à acétylcholinestérase, dont la stabilisation est améliorée.

Un but particulier de la présente invention est d'obtenir une amélioration de la stabilisation à la chaleur (dessiccation), à l'action de protéases, aux variations du pH et/ou de la pression.

Un autre but de l'invention est d'obtenir un procédé d'obtention dudit support qui soit simple, rapide et peu coûteux.

## Eléments caractéristiques de l'invention.

La présente invention concerne un support solide fixant l'acétylcholinestérase et/ou un récepteur de l'acétylcholinestérase, recouvert d'un film protecteur à base de gélatine et/ou d'albumine et à base de tréhalose.

On entend par support solide, toute structure solide sur laquelle un composant à activité biologique est susceptible d'être fixé par des liens covalents, ou des interactions adsorptives.

De tels supports solides peuvent notamment être constitués par une colonne de chromatographie, des cupules de plaques de microtitration, des billes, des gels, des fibres, et/ou tout autre support en verre, en plastique, en métal, en papier et/ou en toute autre matière.

Le film protecteur de gélatine et/ou d'albumine est une structure tridimensionnelle telle qu'un gel, dans lequel un solvant est immobilisé. Ce film est donc constitué d'un réseau tridimensionnel continu de macromolécules ou particules connectées dans une phase liquide continue. Selon l'invention, ce film contient également du tréhalose.

De manière inattendue, la Demanderesse a découvert que le film protecteur selon l'invention assure la stabilisation de l'acétylcholinestérase et/ou d'un récepteur à acétylcholinestérase disposé sur ledit support solide, face à la dessiccation, à l'action de protéases ou aux modifications du pH et/ou de la pression.

On entend par stabilisation le maintien de l'activité enzymatique du ou des sites réactionnels de l'acétylcholinestérase ou du récepteur à acétylcholinestérase.

Le film protecteur selon l'invention est caractérisé par l'effet synergique inattendu de l'action protectrice de la gélatine (et/ou de l'abumine) ainsi que du tréhalose.

D'autres additifs, tels que définis par Gray (Additives and Enzymes Stability, Biocatalyses 1, pp 187-196 (1988)) et Gianfreda et al. (Enzymes Stabilisation and Molecular and Cellular Biochemistry, 100, pp 97-128 (1991)) peuvent également être utilisés pour augmenter cette stabilisation.

Ces additifs peuvent notamment être des alcools polyhydriliques, des solvants organiques, des polymères et/ou des composés ioniques ou non ioniques.

Avantageusement, la stabilisation du support solide selon l'invention s'observe lorsque ledit support est séché, par exemple lorsqu'il est soumis à une dessiccation à des températures élevées (de préférence comprises entre 4 °C et 37 °C, voire jusqu'à des températures proches de 50 °C).

La présente invention concerne également la colonne de chromatographie ou le kit de diagnostic comprenant le support solide selon la présente invention.

Un autre aspect de la présente invention concerne le procédé d'obtention du support solide selon la présente invention, dans lequel on fixe sur un support solide, l'acétylcholinestérase et/ou un récepteur à acétylcholinestérase, on recouvre celui-ci d'un gel, solution comprenant de la gélatine et/ou de l'albumine ainsi que du tréhalose, et on forme sur ledit support solide un film protecteur par évaporation du solvant du gel.

De préférence, la concentration de la gélatine dans la solution est comprise entre 0,5 et 10 mg / ml de solution, de préférence comprise entre 1 et 5 mg / ml de solution.

De préférence, la concentration d'albumine dans la solution est comprise entre 5 et 100 mg / ml de solution, de préférence comprise entre 10 et 50 mg / ml de solution.

Avantageusement, la concentration de tréhalose dans la solution est comprise entre 10 et 150 mg / m de solution, de préférence entre 20 et 50 mg / ml de solution.

La présente invention sera décrite de manière plus détaillée en référence aux exemples suivants donnés à titre d'illustration non limitative de la présente invention.

## Brève description des figures.

La figure 1    représente l'inhibition de l'activité enzymatique de l'acétylcholinestérase (en %) en fonction de doses croissantes de pyridostigmine dans le sérum.

La figure 2    représente l'inhibition de l'activité enzymatique de l'acétylcholinestérase (en %) en fonction de l'augmentation croissante en néostigmine dans le sérum.

La figure 3    représente l'inhibition de l'activité enzymatique de l'acétylcholinestérase (en %) en fonction de l'augmentation croissante en ésérine.

Exemple 1.

De l'acétylcholinestérase extraite du poisson torpille et purifiée par chromatographie d'affinité d'après la méthode de Hirt (Photoaffinity labelling of cholinesterases, Ehret-Sabatier et al. Eur. J. Biochem. 1992, 203, pp 475-481) est mise en solution dans un tampon phosphate 0,05 M à pH 7,2 à une concentration de 2 μg / ml et utilisée à raison de 100 μl par puits pour la sensibilisation de puits de plaques de microtitration. Après une nuit d'incubation à 4 °C, les puits sont rincés puis soumis aux différents traitements suivants :

- 1. blanc (aucun ajout)
- 2. rinçage par une solution d'eau physiologique tamponnée à pH 7 et contenant 15 % en tréhalose;
- 3. recouvrement par une solution d'eau tamponnée à pH 7 par 0,05 M phosphate et contenant 5% de gélatine (240 bloom), puis élimination de la gélatine sans rinçage;
- 4. recouvrement par une solution 5% gélatine contenant 15 % de tréhalose, puis élimination de la gélatine sans rinçage.

La gélatine est solubilisée par chauffage à 47 °C puis refroidie à 37 °C pour sa répartition dans les puits. Les puits des plaque de microtitration ainsi traités sont placés à 4 °C, 37 °C et 50 °C durant 7 jours dans des étuves sèches, puis analysés pour leur activité cholinestérasique, selon la technique décrite par Ellman. Les résultats sont donnés en absorbance lus à 412 nm et sont rapportés dans le tableau 1.

**Tableau 1.**

| Activité enzymatique résiduelle après entreposage de puits sensibilisés par l'acétylcholinestérase à différentes températures durant 7 jours. | | | |
|---|---|---|---|
| | 4 °C | 37 °C | 50 °C |
| 1 Contrôle | 1,72 | 0,42 | 0,05 |
| 2 Tréhalose | 1,88 | 0,97 | 0,12 |
| 3 Gélatine | 1,67 | 1,04 | 0,56 |
| 4 Gél. + Tréh. | 1,79 | 1,82 | 1,71 |

On voit l'extraordinaire amélioration de la stabilité de l'enzyme lorsque les deux facteurs de stabilisation associés, film protecteur et sucre, agissent en synergie.

Exemple 2.

De la peroxydase de raifort (0,2 μg / ml) a été utilisée pour sensibiliser des puits de plaque de microtitration selon la même technique. Après sensibilisation des puits durant une nuit à 4 °C, les puits ont été recouverts soit par une solution chaude (47 °C pour a solubilisation, 37 °C pour la répartition dans les puits) de gélatine à 5 %, soit par une solution d'albumine à 5 %, contenant l'une et l'autre de la tréhalose en concentration décroissante à partir d'une concentration de 8 %. Du triton X100 à une concentration de 0,04 % a été ajouté à la solution d'albumine comme agent mouillant. Les puits ont été immédiatement vidés sans rinçage, et ont ensuite été placés à 4 °C et à 37 °C durant 7 jours dans une étuve sèche. L'activité peroxydasique résiduelle a été mesuré par addition de 100 μl d'une solution de tétraméthylbenzidine et $H_2O_2$ dans un tampon citrate-phosphate à pH 5,5. La réaction colorée bleue obtenue a été transformée en une couleur jaune sous l'action de l'acide sulfurique 0,5 N ajouté pour arrêter la réaction enzymatique. Les résultats sont donnés en absorbance lus à 450 nm et sont rapportés dans le tableau 2.

**Tableau 2.**

| Tréhalose | Gélatine | | Albumine | |
|---|---|---|---|---|
| mg / ml | 4 °C | 37 °C | 4 °C | 37 °C |
| 8,0 | 1,86 | 1,69 | 1,78 | 1,81 |
| 4,0 | 1,78 | 1,74 | 1,79 | 1,73 |
| 2,0 | 1,82 | 1,69 | 1,84 | 1,69 |
| 1,0 | 1,42 | 0,97 | 1,78 | 1,74 |
| 0,5 | 1,13 | 0,48 | 1,67 | 1,43 |
| 0 | 0,97 | 0,36 | 1,37 | 0,52 |

On voit que la protection accordée par le tréhalose incorporé dans le film protecteur est sensiblement la même pour l'albumine et la gélatine, avec un léger avantage pour l'albumine, sans doute parce que contrairement à la gélatine, ce film protéinique ne se ramollit pas à 37 °C. Deux pour cent de tréhalose inclus dans la solution gélifiante semble être nécessaire et suffisants pour stabiliser l'enzyme immobilisée sur la phase solide.

Exemple 3.

Une analyse plus détaillée de la concentration en gélatine et en tréhalose nécessaires pour obtenir une protection optimale de l'acétylcholinestérase de poisson torpille immobilisée et séchée dans des puits de plaques de microtitration est donnée dans le tableau 3.

## Tableau 3.

| Stabilité : % résiduel d'activité | | | | | | | | |
|---|---|---|---|---|---|---|---|---|
| Protection | Jour 10 | | | | Jour 31 | | | |
| | 4°C | T.A. | 37°C | 50°C | 4°C | T.A. | 37°C | 50°C |
| 0 % de gélatine | | | | | | | | |
| Tréhalose    0% | 0 | 0 | 0 | 0 | | | | |
| 2% | 27 | 22 | 0 | 17 | | | | |
| 5% | 48 | 29 | 8 | 35 | | | | |
| 10% | 69 | 72 | 26 | 44 | | | | |
| 15% | 73 | 76 | 43 | 65 | | | | |
| 0,5 % de gélatine | | | | | | | | |
| Tréhalose    0% | 100 | 100 | 0 | – | 100 | 100 | 0 | 43 |
| 2% | 100 | 100 | 72 | 100 | 100 | 100 | 21 | 100 |
| 5% | 100 | 100 | 100 | 100 | 100 | 100 | 70 | 100 |
| 10% | 100 | 100 | 100 | 100 | 100 | 100 | 69 | 100 |
| 15% | 100 | 100 | 100 | 100 | 100 | 100 | 67 | 100 |

De l'acétylcholinestérase de poisson torpille a été fixée dans les puits d'une plaque de microtitration selon la méthode décrite dans l'exemple 1. Après lavage, les puits ont été immergés pendant 12 heures à 4 °C dans des solutions de tréhalose, en présence ou absence de 0,5 % de gélatine. Les puits ont ensuite été vidés, et l'activité enzymatique de l'acétylcholinestérase a été mesurée selon la méthode décrite dans l'exemple 1. Les puits ont ensuite été maintenus à une température de 4 °C, température ambiante, 37 °C et 50 °C durant plusieurs jours. L'activité résiduelle a été mesurée après 10 jours et 31 jours de stockage.

On voit qu l'enzyme non protégée par du tréhalose ou de la gélatine, séchée dans les puits d'une plaque de microtitration, est détruite après 10 jours de stockage à 4 °C. Des concentrations croissantes de tréhalose protègent l'activité enzymatique. Cependant, même avec 150 mg tréhalose / ml de solution, 27 % de l'activité enzymatique par rapport aux 100 % de l'activité de départ de l'enzyme a disparu après 10 jours de stockage à 4 °C.

La gélatine seule protège l'activité enzymatique à température ambiante et à 4 °C mais pas à 37 °C (mesurée après 10 jours de stockage) et très partiellement après 31 jours de stockage à 50 °C. Une concentration de 50 mg tréhalose / ml de solution solubilisé dans la solution de gélatine donne une bonne protection. Il faut noter que la protection est meilleure à 50 °C qu'à 37 °C, probablement parce que la gélatine sèche immédiatement à 50 °C dans une étuve sèche mais passe à l'état liquide à 37 °C. Ce phénomène n'a pas été systématiquement observé et est mis en relation avec l'épaisseur de la couche de gélatine, variable d'après la durée et la température de stockage.

Exemple 4.

Les acétylcholinestérases sont d'origines variées (espèces animales différentes) et se retrouvent dans des organes différents. Une analyse de la résistance des acétylcholinestérases de congre et de poisson torpille, en présence d'albumine, d'albumine plus tréhalose et de gélatine plus tréhalose montre que toutes les acétylcholinestérases ne sont pas également résistantes (tableau 4). La sensibilisation des puits de plaques de microtitration et l'analyse enzymatique ont eu lieu selon les protocoles décrits dans l'exemple 1.

L'albumine plus tréhalose ne protège pas aussi bien que la gélatine plus tréhalose (voir 50 jours de stockage à 50 °C) et l'absence de tréhalose se fait plus nettement sentir avec l'acétylcholinestérase de poisson torpille (29 % d'activité résiduelle) versus l'acétylcholinestérase de congre (60 % d'activité résiduelle)

**Tableau 4.**

| Stabilité de deux acétylcholinestérases différentes : % résiduel d'activité | | | | | | | |
|---|---|---|---|---|---|---|---|
| JOURS | TEMP. | Albumine 5 % | | Albumine 5 % | | Gélatine 0,5 % | |
| | | Tréhalose 0 % | | Tréhalose 5 % | | Tréhalose 5 % | |
| | | Cong | Torp | Cong | Torp | Cong | Torp |
| 0 | | 100 | 100 | 100 | 100 | 100 | 100 |
| 6 | 37 °C | 100 | 91 | 100 | 100 | 100 | 100 |
| | 50 °C | 96 | 81 | 100 | 100 | 100 | 100 |
| 14 | 37 °C | 100 | 88 | 100 | 100 | 100 | 100 |
| | 50 °C | 88 | 75 | 100 | 100 | 100 | 100 |
| 30 | 37 °C | 100 | 89 | 100 | 100 | 100 | 100 |
| | 50 °C | 88 | 46 | 100 | 100 | 100 | 100 |
| 50 | 37 °C | 86 | 63 | 100 | 100 | 100 | 100 |
| | 50 °C | 60 | 29 | 96 | 90 | 100 | 100 |

Exemple 5.

L'eau n'est normalement pas tamponnée et a, normalement, un pH de 5,5 en raison du $CO_2$ dissous. L'eau, analysée sans précautions particulières pour la présence de pesticides, peut présenter des extrêmes de pH qui peuvent influer sur l'activité enzymatique de l'acétylcholinestérase, dont l'optimum se situe aux environs de 7,2. De même, les pesticides sont normalement dissous dans le cyclohexane, qui peut influer sur la réaction.

L'activité enzymatique de l'acétylcholinestérase de poisson torpille a été vérifiée après exposition durant 18 heures à 4 °C à de l'eau où le pH a été varié par des tampons (phosphate, acétate et $NaHCO_3$) 0.005 M. De même, une analyse a été effectuée après traitement durant 18 heures à 4 °C par du cyclohexane pur, de puits sensibilisés par l'enzyme. On voit dans le tableau 5 que la gélatine plus le tréhalose protège efficacement jusqu'à un pH de 5 et de 8,2. L'activité de l'enzyme protégée est maintenue malgré un contact avec du cyclohexane.

**Tableau 5.**

| pH | Activité relative | |
|---|---|---|
| | Non traité | 0,5 % gélatine<br>5 % tréhalose |
| 3 | 07 | 08 |
| 4 | 39 | 80 |
| 5 | 40 | 100 |
| 6 | 44 | 100 |
| 7.2 | 100 | 100 |
| 8.2 | 100 | 100 |
| 9.5 | 13 | 40 |
| 11 | 02 | 02 |
| Cyclohexane | 38 | 100 |

Exemple 6.

L'acétylcholinestérase de congre a été utilisée pour sensibiliser des tubes en verre. L'avantage d'une telle sensibilisation en tubes au lieu de cupules de plaques de microtitration réside dans l'augmentation de la surface sensibilisée, qui, à son tour, peut potentiellement augmenter la sensibilité des mesures effectuées. 2 ml d'une solution d'enzyme ont été utilisés par tube pour la sensibilisation, selon le protocole décrit dans l'exemple 1. Après sensibilisation, l'enzyme fixée a été protégée par introduction dans les tubes de 2 ml d'une solution 0,2 % de gélatine contenant 5 % de tréhalose. Après 2 heures à 4 °C, les tubes ont été vidés et stockés durant 4 jours à différentes températures avant vérification de l'activité enzymatique résiduelle, selon le protocole décrit dans l'exemple 1. Le tableau 6 donne les résultats de cette analyse.

On voit que la protection conférée par la gélatine contenant du tréhalose est substantielle, sans pour cela atteindre l'efficacité observée avec des puits de plaques de microtitration.

**Tableau 6.**

| % d'activité enzymatique résiduelle | | |
|---|---|---|
| | Non protégé | Protégé |
| Temps 0 | 100 | – |
| Temps 4 jours | | |
| 4 °C | 19 | 97 |
| T.A. | 13 | 61 |
| 37 °C | 10 | 81 |
| 50 °C | 8 | 81 |

Exemple 7.

L'analyse d'un pesticide courant, le Naled, solubilisé dans l'eau aux concentrations 10, 5, 2.5, 1 et 0 µg / litre a été effectuée avec une enzyme acétylcholinestérase de congre sensibilisant des puits de plaques de microtitration et protégé par le tréhalose plus des concentrations variés de gélatine, d'albumine, de gélatine plus albumine, selon les méthodes décrites supra.

Les résultats obtenus (tableau 7) en suivant les protocoles d'analyse décrits dans l'exemple 1 montrent que la sensibilité du test de détection augmente avec la concentration décroissante de polymère, la sensibilité la moins satisfaisante étant observée avec un mélange de gélatine et albumine. Dans le test, la plus grande sensibilité est obtenue avec 0,1 % de gélatine.

## Tableau 7.

| % résiduel d'activité | | | | | |
|---|---|---|---|---|---|
| Protection : | NALED (µg / litre) | | | | |
| | 10 | 5 | 2 | 1 | 0 |
| 5 % de tréhalose plus | | | | | |
| 0,1% gélatine | 30 | 50 | 73 | 78 | 100 |
| 0,2% gélatine | 29 | 60 | 80 | 83 | 100 |
| 0,4% gélatine | 36 | 57 | 81 | 88 | 100 |
| 1 % albumine | 50 | 67 | 82 | 88 | 100 |
| 2 % albumine | 58 | 83 | 82 | 88 | 100 |
| 4 % albumine | 69 | 85 | 94 | 96 | 100 |
| | | | | | |
| 1 % d'albumine plus | | | | | |
| 0,1 % gélatine | 52 | 100 | 100 | 100 | 1 |
| 0,2 % gélatine | 68 | 99 | 100 | 100 | 100 |

## Exemple 8.

Cinq pesticides (le Naled, le Paraoxon, le Dichlorvos, le Carbofuran et le Carbaryl) ont été analysés à différentes concentrations sur des plaques de microtitration sensibilisées à l'acétylcholinestérase de poisson torpille selon la méthode décrite, protégées ensuite par de la gélatine - tréhalose et utilisées comme décrit dans l'exemple 1. On voit dans le tableau 8 que la sensibilité n'est pas identique pour tous : le test est plus sensible pour le Naled et le Carbofuran, suivis de près par le Paraoxon. Le Dichlorvos et le Carbaryl sont détectés avec une sensibilité 100 fois moindre.

**Tableau 8.**

| Pesticides (µg / l) | | Activité résiduelle (%) |
|---|---|---|
| Naled | 1000 | 0 |
| | 100 | 0 |
| | 50 | 0 |
| | 10 | 11 |
| | 1 | 73 |
| Paraoxon éthyl | 1000 | 0 |
| | 100 | 0 |
| | 50 | 0 |
| | 10 | 40 |
| | 1 | 80 |
| Dichlorvos | 1000 | 3 |
| | 100 | 49 |
| | 50 | 60 |
| | 10 | 80 |
| Carbofuran | 1000 | 14 |
| | 100 | 18 |
| | 50 | 20 |
| | 10 | 35 |
| | 1 | 73 |
| Carbaryl | 1000 | 18 |
| | 100 | 44 |
| | 50 | 61 |
| | 10 | 80 |

## Exemple 9.

Le Naled et le Carbaryl ont été dilués à concentrations variées dans un extrait de salades. L'analyse de la présence dans inhibiteurs a été effectuée selon le protocole décrit dans l'exemple 1. On voit dans le tableau 9 que la sensibilité des tests est semblable à celle obtenue lorsque les pesticides sont analysés dans l'eau.

**Tableau 9.**

| Pesticides (µg / l) | | Activité relative (%) |
|---|---|---|
| Naled | 1000 | 3 |
| | 500 | 9 |
| | 100 | 20 |
| | 50 | 63 |
| | 10 | 90 |
| Carbaryl | 1000 | 28 |
| | 500 | 26 |
| | 100 | 51 |
| | 50 | 61 |
| | 10 | 80 |

Exemple 10.

La pyridostygmine est un médicament utilisé pour le traitement de la myasthénie. Sa détermination dans le sérum de malades sous traitement s'impose lorsque des doses maximales ou subliminaires en sont administrées.

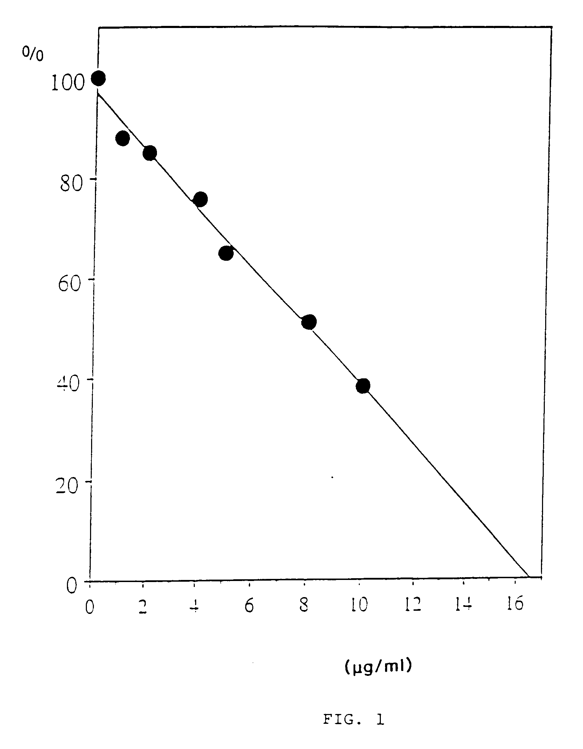

Un sérum de sujet non malade a été additionné de concentrations croissantes de pyridostygmine puis analysé pour l'activité enzymatique de l'acétylcholinestérase selon le protocole déjà défini dans les exemples précédents. On voit dans la figure 1 que le test de détection de cet inhibiteur de l'acétylcholinestérase rend compte de la présence de 1 à 2 µg / ml de ce produit dans le sérum.

Exemple 11.

La néostygmine est un médicament utilisé pour le traitement de la myasthénie. Sa détermination dans le sérum de malades sous traitement s'impose lorsque des doses maximales ou subliminaires en sont administrées.

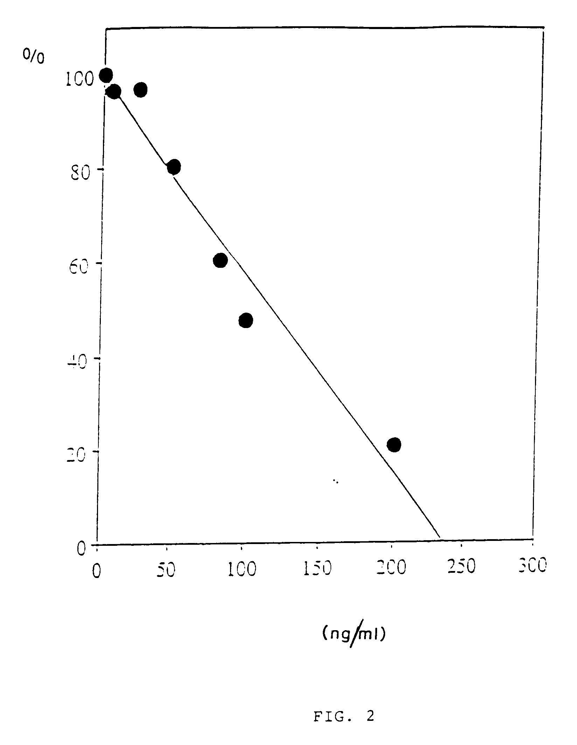

Un sérum de sujet non malade a été additionné de concentrations croissantes de néostygmine puis analysé pour l'activité enzymatique de l'acétylcholinestérase selon le protocole déjà défini dans les exemples précédents. On voit dans la figure 2 que le test de détection de cet inhibiteur de l'acétylcholinestérase rend compte de la présence de 50 ng / ml de ce produit dans le sérum.

Exemple 12.

L'ésérine est un médicament utilisé pour le traitement de la maladie d'Alzheimer. Sa détermination dans le sérum de malades sous traitement s'impose lorsque des doses maximales ou subliminaires en sont administrées.

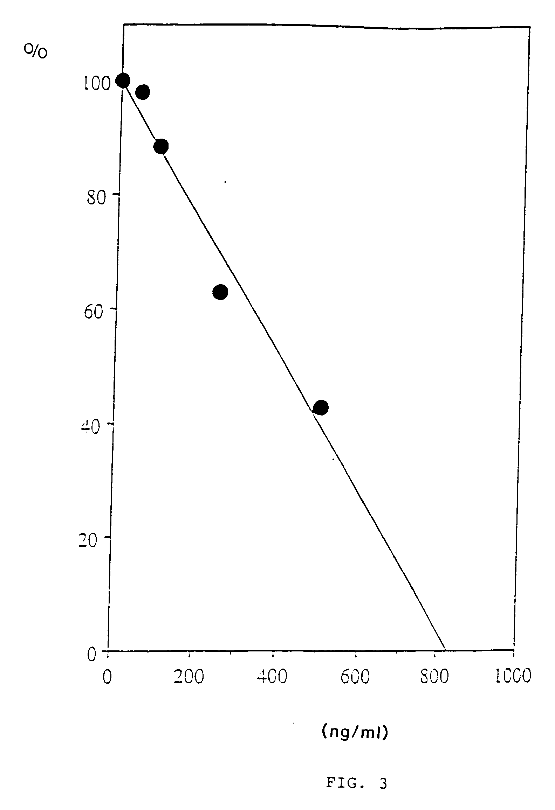

Un sérum de sujet non malade a été additionné de concentrations croissantes d'ésérine puis analysé pour l'activité enzymatique de l'acétylcholinestérase selon le protocole déjà défini dans les exemples précédents. On voit dans la figure 3 que le test de détection de cet inhibiteur de l'acétylcholinestérase rend compte de la présence de 100 ng / ml de ce produit dans le sérum.

**Revendications**

1. Support solide fixant l'acétylcholinestérase et/ou un récepteur à acétylcholinestérase, recouvert d'un film protecteur à base de gélatine et/ou d'albumine et à base de tréhalose.

2. Support selon la revendication 1, caractérisé en ce qu'il est séché.

3. Colonne de chromatographie comprenant le support solide selon la revendication 1 ou 2.

4. Trousse de diagnostic comprenant le support selon la revendication 1 ou 2.

5. Procédé d'obtention du support selon la revendication 1 ou 2, caractérisé en ce que l'on fixe sur un support solide une acétylcholinestérase et/ou un récepteur à acétylcholinestérase, en ce qu'on recouvre ledit support d'un gel comprenant de la gélatine et du tréhalose, et en ce qu'on forme sur ledit support solide un film protecteur par évaporation du solvant du gel.

FIG. 1

FIG. 2

FIG. 3

**Office européen des brevets**

# RAPPORT DE RECHERCHE EUROPEENNE

Numero de la demande

EP 95 87 0021

## DOCUMENTS CONSIDERES COMME PERTINENTS

| Catégorie | Citation du document avec indication, en cas de besoin, des parties pertinentes | Revendication concernée | CLASSEMENT DE LA DEMANDE (Int.Cl.6) |
|---|---|---|---|
| D,Y | FR-A-2 677 373 (ANADIAG)<br>* page 6, ligne 6 - page 9, ligne 16 *<br>--- | 1-5 | C12N9/96<br>C12N11/00<br>G01N33/543 |
| D,Y | EP-A-0 140 489 (WAKO PURE CHEMICAL INDUSTRIES, LTD.)<br>* le document en entier *<br>--- | 1-5 | |
| D,Y | EP-A-0 192 320 (UNILEVER PLC.)<br>* le document en entier *<br>----- | 1-5 | |

**DOMAINES TECHNIQUES RECHERCHES (Int.Cl.6)**

C12N
G01N

Le présent rapport a été établi pour toutes les revendications

| Lieu de la recherche | Date d'achèvement de la recherche | Examinateur |
|---|---|---|
| LA HAYE | 3 Juillet 1995 | Griffith, G |

**CATEGORIE DES DOCUMENTS CITES**

X : particulièrement pertinent à lui seul
Y : particulièrement pertinent en combinaison avec un autre document de la même catégorie
A : arrière-plan technologique
O : divulgation non-écrite
P : document intercalaire

T : théorie ou principe à la base de l'invention
E : document de brevet antérieur, mais publié à la date de dépôt ou après cette date
D : cité dans la demande
L : cité pour d'autres raisons

& : membre de la même famille, document correspondant

EPO FORM 1503 03.82 (P04C02)